# EUROPÄISCHE PATENTSCHRIFT

(11) **EP 1 360 942 B1**
(45) Veröffentlichungstag und Bekanntmachung des Hinweises auf die Patenterteilung: **14.12.2005**
(21) Anmeldenummer: 03009094.8
(22) Anmeldetag: 19.04.2003
(51) Int. Cl.: A61F 2/06, A61F 2/04

(54) **Stent**
Stent
Stent

(30) Priorität: 11.05.2002 DE 10221076
(43) Veröffentlichungstag der Anmeldung: 12.11.2003
(73) Patentinhaber: Willy Rüsch GmbH, 71394 Kernen-Rommelshausen (DE)
(72) Erfinder: Schlick, Tilman, 73728 Esslingen (DE); KUHNLE, Markus, 73660 Urbach (DE)
(74) Vertreter: Kohler Schmid Möbus

(56) Entgegenhaltungen:
- EP-A- 0 808 614
- EP-A- 0 818 184
- EP-A- 1 264 582
- EP-B- 0 901 353
- WO-A-00/32137
- SU-A- 1 292 761

## Beschreibung

Die Erfindung betrifft einen Stent zur Schienung und/oder zum Offenhalten eines Hohlorgans, bestehend aus einem tubulären Körper, der einen Durchmesser aufweist, der durch axiale Verschiebung der Enden des Körpers relativ zueinander veränderbar ist, wie dieser Stent im Oberbegriff des Anspruchs 1 definiert wird. Ein solcher stent ist aus der Druckschrift EP 0 901 353 B1 bekannt.

Mit dem bekannten Stent können Gefäßverengungen nicht nur radial aufgeweitet sondern auch geschient bzw. offengehalten werden. In einem verjüngten und gelängten Zustand wird der Stent in ein Hohlorgan eingeführt und an der verengten Stelle des Hohlorgans radial expandiert, damit das Hohlorgan an dieser Stelle möglichst dauerhaft sein ursprüngliches Lumen einnimmt. Zur Selbstexpansion sind Geflechte bekannt, die man zu Stents verarbeitet. Die Geflechte können gegen ihre belastungsfreie Ausgangsstruktur gelängt werden. Gibt man die Längung bzw. eine radiale Deformation auf, so kehren diese bekannten Stents in ihren Ausgangszustand zurück, indem sie sich radial weiten. Diesen Effekt nützt man zur Weitung von Stenosen in Hohlorganen, so dass deren Funktion möglichst nicht beeinträchtigt wird.

Die EP 0 808 614 A2 offenbart einen Stent mit tubulären Stentabschnitten umfassend zylindrische, radiale elastische Einheiten mit einer "zig-zag" Form die mittels eines aus einem elastischen Film hergestellten Umhüllungs-Fixierungs-Teils miteinander verbunden sind.

Aufgabe der Erfindung ist es, einen Stent zu schaffen, der Bewegungen der Hohlorgane lagestabil verbessert aufnehmen und weiterleiten kann.

Erfindungsgemäß wird die Aufgabe durch die Merkmale des Patentanspruchs 1 gelöst.

Der erfindungsgemäße Stent hat damit den wesentlichen Vorteil, dass er in longitudinaler Richtung Abschnitte mit unterschiedlich starken Rückstellkräften aufweisen kann. Jeder Stentabschnitt kann ein eigenständiges Design und/oder Bewegungsverhalten aufweisen, so dass Stents mit reduziertem Gesamtvorshortening (axiale Längung der freien Stentenden bei radialer Kompression eines Stents) und speziellen migrationshemmenden Eigenschaften geschaffen werden können. Dadurch dass die Stentabschnitte über gewebefreie elastische tubuläre Abschnitte miteinander verbunden sind, bleiben die einzelnen Stentabschnitte hoch beweglich und können sich somit Hohlorganverläufen verbessert anpassen. Die elastischen tubulären Abschnitte (dünne hochbewegliche reißfeste Kunststofffolien) lassen eine relative Bewegung der einzelnen Stentabschnitte untereinander zu (mehr oder weniger starke Invagination, die von der gewählten Wandstärke und dem ausgewählten Material für die elastischen tubulären Abschnitte abhängig ist). Der erfindungsgemäße Stent passt sich, ohne zu migrieren, gegebenenfalls einer Peristaltik von Hohlorganen an. Besondere, neue Stenteigenschaften leiten sich aus der Beabstandung, Teilung der einzelnen Stentabschnitte zueinander in Verbindung mit den elastischen tubulären Abschnitten ab, die die einzelnen Stentabschnitte untereinander verbinden.

Der erfindungsgemäße Stent ist mittels Hilfsmitteln expandierbar oder er kann als selbstexpandierbarer Stent ausgelegt sein.

Die erfindungsgemäßen Stentabschnitte weisen ein gewobenes, geflochtenes tubuläres Geflecht und/oder eine oder mehrere phasenverschobene Wendeln in einer oder mehreren Ebenen auf.

Dies hat den Vorteil, dass unterschiedliche Stützkonstruktionen in den einzelnen Stentabschnitten in ein und demselben Stent eingesetzt werden können. Über unterschiedliche Stützkonstruktionen oder über unterschiedlich stark ausgeprägte Stützkonstruktionen lassen sich das Bewegungs- und das Verformungsverhalten des erfindungsgemäßen Stents definiert steuern.

Der Körper des erfindungsgemäßen Stents ist in belastungsfreiem expandiertem Zustand aus einem ersten Stentabschnitt mit einem ersten Durchmesser, einem zweiten und dritten Stentabschnitt mit einem zweiten und dritten Durchmesser und aus zwei elastischen tubulären Abschnitten gebildet, die den ersten, zweiten und dritten Stentabschnitt miteinander verbinden.

Dies hat den Vorteil, dass mit einem dreiteiligen Stent eine Hohlorganauskleidung geschaffen werden kann, die es beispielsweise einem angrenzenden Tumor erlaubt, elastisch tubuläre Abschnitte eines erfindungsgemäßen Stents in das Lumen des Stents zu protrudieren Die Abstützung und Fix ierung eines Stents in der Stenose wird dadurch weiter verbessert (Migrationsschutz).

In weiterer Ausgestaltung der Erfindung sind die Durchmesser der Stentabschnitte unterschiedlich. Dies hat den Vorteil, dass unterschiedlich große Rückstellkräfte ausgeprägt lokalbezogen ausgebildet werden können. Die elastischen tubulären Abschnitte passen sich Querschnittsveränderungen längs eines Stents problemlos an und im belastungsfreien Zustand ist ein ansatzloser faltenfreier Übergang von einem kleineren Stentabschnitt auf einen größeren Stentabschnitt und umgekehrt möglich.

In einer weiteren Ausführungsform des erfindungsgemäßen Stents weist bei mehreren Wendeln in mehreren Ebenen mindestens eine Wendel eine entgegengesetzte Windungsrichtung auf. Dies hat den Vorteil, dass der erfindungsgemäße Stent eine reversible Torquierung erhält, so dass sich bei einem Expansionsvorgang die Vortorquierung zurückstellt und sich der erfindungsgemäße Stent schraubenartig an die Innenoberfläche eines Hohlorgans anlegt. Dieser sogenannte "Torquierungseffekt" lässt sich je nach Bedarf in jedem der einzelnen Stentabschnitte verwirklichen.

Bevorzugt ist weiterhin, dass die elastischen tubulären Abschnitte aus einem gewebeverträglichen Kunststoff hergestellt sind und/oder die gewebefreien elastischen tubulären Abschnitte im belastungsfreien Zustand unterschiedliche Durchmesser aufweisen.

Dies hat den Vorteil, dass der erfindungsgemäße Stent im platzierten Zustand im Bereich seiner beiden Enden Gewebereizungen weitgehend verhindert oder abschwächt, und es ist möglich, auch die Stützkonstruktionen (Geflechte, Wendeln) mit einer Beschichtung oder Beschichtungen zu überziehen, die die elastischen tubulären Abschnitte bilden. Unterschiedliche Durchmesser der Stentabschnitte längs eines Stents sind dabei kein Hindernis.

In einer besonders bevorzugten Ausführungsform des erfindungsgemäßen Stents weist der Körper, im Querschnitt gesehen, im ersten, zweiten und dritten Stentabschnitt ein Geflecht und/oder eine oder mehrere Wendeln auf, die von einer oder mehreren elastischen Kunststoffschichten ummantelt sind, die materialschlüssig und geflecht- und wendelfrei in die elastischen tubulären Abschnitte übergehen.

Dies hat den Vorteil, dass die Innenoberfläche eines erfindungsgemäßen Stents, je nach Bedarf, besonders glatt und/oder beispielsweise hydrophil beschichtet ausgebildet sein kann, und die im Außenumfang des Stents ausgebildete Ummantelung mit einer Kunststoffschicht fixiert zum einen das Geflecht oder die Wendeln umgeben und zum anderen die äußere und innere Kunststoffbeschichtung längs des erfindungsgemäßen Stents materialschlüssig in die elastischen tubulären Abschnitte übergehen kann. Mit dieser Ausführungsform werden besonders widerstandsfähige Stents geschaffen.

Weiterhin kann der Körper eines erfindungsgemäßen Stents in axialer Richtung in der Wandung ein Filament aufweisen, das zum einen die Funktion eines Sicherungsfadens haben kann, der gewährleistet, dass die unterschiedlichen Stentabschnitte auch bei stärkerer Belastung zusammengehalten werden und zum anderen kann dieser Faden röntgenschattengebend ausgelegt sein. Dies hat den Vorteil, dass auch nach einer zeitlich längeren Platzierung die Lage des Stents im Hohlorgan exakt geprüft, bestimmt und nachgewiesen werden kann. Es versteht sich, dass auch mehrere Filamente, z. B. an zwei gegenüberliegenden Seiten, oder in allen vier Querschnittsquadranten in die Wandung eines Stents eingearbeitet sein können.

Das längs des Stents eingearbeitete Filament kann auch über den Stent als Sicherungsfaden hinausragen und dient dann als Zugfaden oder zum Befestigen des erfindungsgemäßen Stents. Mehrere Filamente können, über den Umfang des Stents gesehen, in die Wandung des Stents eingearbeitet sein.

In einer weiteren Ausgestaltung der Erfindung ist im proximalen Endbereich des Körpers des erfindungsgemäßen Stents, über den Umfang des Körpers gesehen, ein Faden eingearbeitet, dessen Länge über den Stent hinausragt. Dies hat den Vorteil, dass mit einem derartigen Faden ein Fadenringanker, ähnlich einer Tabaksbeutelnaht, geschaffen werden kann, der die Entnahme eines platzierten Stents erleichtert. Durch ein tabakbeutelartiges Zusammenziehen des proximalen Stentendes lässt sich der erfindungsgemäße Stent vom angrenzenden Hohlorgangewebe lösen und über den Faden aus dem Hohlorgan herausziehen.

In einer weiteren Ausführungsform ist am distalen Ende des Körpers ein Rückschlagventil, insbesondere ein Folienventil, ausgebildet, das den Vorteil hat, insbesondere bei der Platzierung des erfindungsgemäßen Stents im Ösophagus, dass Flüssigkeit und sonstige Ernährungsstoffe nur in einer Richtung strömen können. Das als Refluxventil ausgebildete Folienventil besteht aus zwei dünnen Folienblättchen, die beispielsweise an den Rändern miteinander verschweißt sind.

Gewebefrei bedeutet auch zugleich geflecht- oder wendelfrei.

Weitere Vorteile der Erfindung ergeben sich aus der Figurenbeschreibung. Die vorstehend genannten und die noch weiter ausgeführten Merkmale können jeweils einzeln oder in beliebigen Kombinationen miteinander erfindungsgemäß verwendet werden. Die erwähnten Ausführungsformen sind nicht als abschließende Aufzählung zu verstehen, sondern haben vielmehr beispielhaften Charakter. Es zeigt:
- Fig. 1: eine erste Ausführungsform eines erfindungsgemäßen Stents in Seitenansicht;
- Fig. 2: eine zweite Ausführungsform eines erfindungsgemäßen Stents in Seitenansicht;
- Fig. 3: eine dritte Ausführungsform eines erfindungsgemäßen Stents in Seitenansicht; und
- Fig. 4: eine vierte Ausführungsform eines erfindungsgemäßen Stents in Seitenansicht.

In den Fign. ist der erfindungsgemäße Stent stark schematisiert dargestellt.

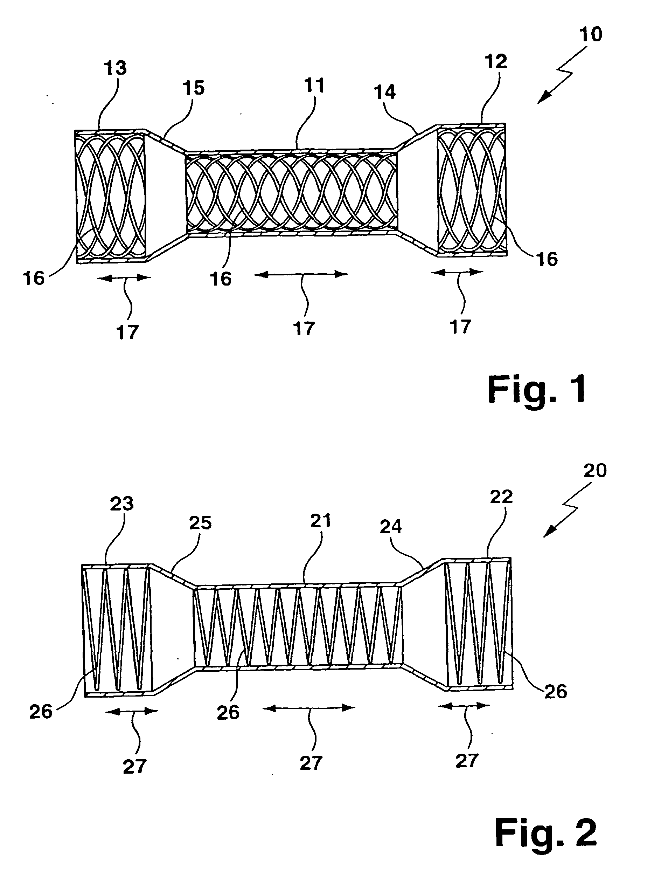

**Fig. 1** zeigt einen Stent 10, der aus einem ersten Stentabschnitt 11, einem zweiten Stentabschnitt 12, einem dritten Stentabschnitt 13, einem ersten elastischen tubulären Abschnitt 14 und einem zweiten elastischen tubulären Abschnitt 15 gebildet ist. Der Stent 10 ist in einem belastungsfreien expandierten Zustand gezeigt. Als Stützkonstruktion ist in den Stentabschnitten 11, 12, 13 ein Geflecht 16 gewählt, das tubulär geflochten oder gewoben sein kann. Die einzelnen Filamente des Geflechts können aus Metall, Kunststoff oder Karbon hergestellt sein. Der erste Stentabschnitt 11 kann das tubuläre Geflecht 16 selbst sein, oder das Geflecht 16 ist zusätzlich noch mit einem Kunststoffmaterial ummantelt. Die Freiräume (Maschen) des Geflechts 16 können offen oder geschlossen sein. Der zweite und dritte Stentabschnitt 12, 13 weisen einen größeren Durchmesser auf als der erste Stentabschnitt 11. Als Stützkonstruktion wurde in den Stentabschnitten 12, 13 ebenfalls das Geflecht 16 des ersten Stentabschnitts 11 gewählt. Die einzelnen Filamente des Geflechts 16 können in den Stentabschnitten 12, 13 unterschiedliche Filamentdurchmesser gegenüber den im ersten Stentabschnitt 11 verwendeten Filamenten aufweisen.

Die Stentabschnitte 11, 12, 13 sind über einen ersten und zweiten elastischen tubulären Abschnitt 14, 15 miteinander verbunden. Die elastischen tubulären Abschnitte 14, 15 sind aus einem beweglichen dünnen Kunststoffmaterial, wie Silikon, hergestellt und verbinden dauerhaft und sicher die Stentabschnitte 11, 12, 13 untereinander. Die elastischen tubulären Abschnitte 14, 15 können Durchmesserveränderungen zwischen den einzelnen Stentabschnitten 11, 12, 13 sicher überbrücken und können sich an die Oberflächenkontur in einem Hohlorgan ohne Freiräume zu bilden anpassen. In Pfeilrichtungen 17 kann der Stent 10 gelängt werden und sich nach einer Längung wieder verkürzen. Es handelt sich hier um einen selbstexpandierbaren Stent 10, der im gelängten Zustand einen kleineren Durchmesser einnimmt als im expandierten Zustand, wie er in der Fig. gezeigt ist.

**Fig. 2** zeigt einen Stent 20, bestehend aus einem ersten Stentabschnitt 21, einem zweiten Stentabschnitt 22, einem dritten Stentabschnitt 23 und einem ersten und zweiten elastischen tubulären Abschnitt 24, 25. Über die elastischen tubulären Abschnitte 24, 25 sind die Stentabschnitte 21, 22, 23 untereinander untrennbar verbunden. Die Stentabschnitte 21, 22, 23 weisen als Stützkonstruktion eine Wendel 26 auf, die in ein dehnbares elastisches Kunststoffmaterial eingebettet ist. In die elastischen tubulären Abschnitte 24, 25 sind keine Armierungsmaterialien eingearbeitet, so dass die Stentabschnitte 21, 22, 23 untereinander und gegeneinander beweglich sind. In Pfeilrichtungen 27 lassen sich die Stentabschnitte 21, 22, 23 längen und verkürzen. Die elastischen tubulären Abschnitte 24, 25 folgen den Bewegungen der Stentabschnitte 21, 22, 23.

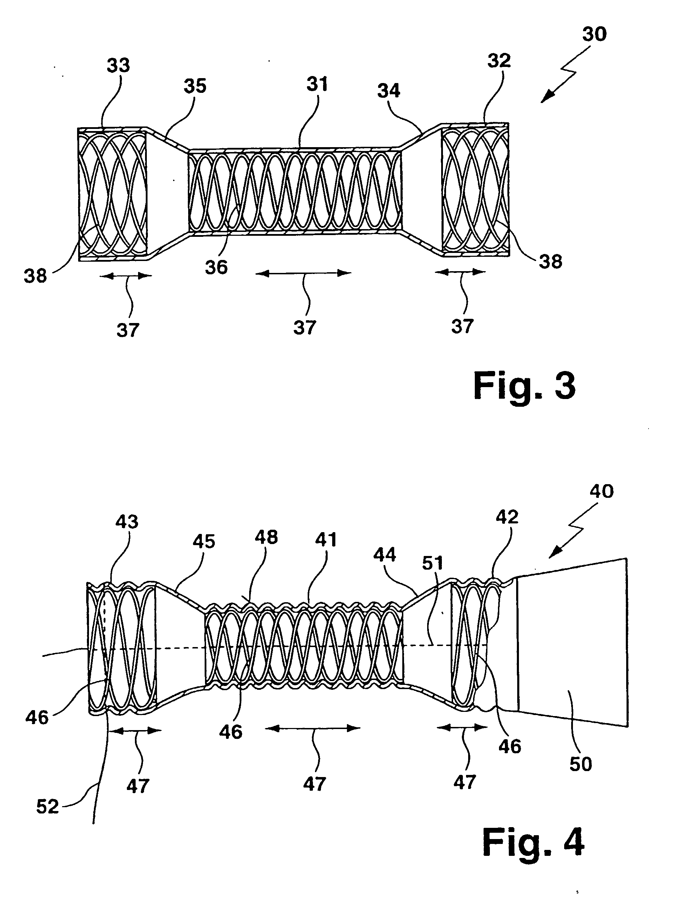

In **Fig. 3** ist ein Stent 30 mit einem ersten Stentabschnitt 31, einem zweiten Stentabschnitt 32 und einem dritten Stentabschnitt 33 gezeigt. Die Stentabschnitte 31, 32, 33 sind über einen ersten und einen zweiten elastischen tubulären Abschnitt 34, 35 miteinander dauerhaft verbunden.

Im ersten Stentabschnitt 31 ist als Stützkonstruktion eine erste und zweite Wendel 36 gewählt, die gegenläufige Windungsrichtungen aufweisen. In einem Kunststoffmaterial eingebettet, ist in einer ersten Ebene im ersten Stentabschnitt 31 eine Wendel 36 angeordnet, und in einer zweiten Ebene verläuft ebenfalls eine Wendel 36, die eine entgegengesetzte Windungsrichtung aufweist. In den Stentabschnitten 32, 33 wurde als Stützkonstruktion ein Geflecht 38 gewählt, das über die elastischen tubulären Abschnitte 34, 35 an den ersten Stentabschnitt 31 angebunden ist. Der Stent 30 lässt sich in Pfeilrichtungen 37 längen und verkürzen.

In **Fig. 4** ist ein weiterer erfindungsgemäßer Stent 40 in Seitenansicht gezeigt, der sich aus einem ersten Stentabschnitt 41, einem zweiten Stentabschnitt 42, einem dritten Stentabschnitt 43, einem ersten elastischen tubulären Abschnitt 44 und einem zweiten elastischen tubulären Abschnitt 45 zusammensetzt. Als Stützkonstruktion wurden gegenläufige Wendeln 46, in zwei verschiedenen Ebenen verlaufend, gewählt. Die Wendeln 46 sind in ein Kunststoffmaterial eingebettet, das ebenfalls für die elastischen tubulären Abschnitte 44, 45 verwendet wird. Die elastischen tubulären Abschnitte 44, 45 sind wendelfrei. In Pfeilrichtungen 47 kann der Stent 40 gelängt und anschließend in radialer Richtung expandiert und in longitudinaler Richtung verkürzt werden. Die Kunststoffbeschichtung der Wendeln 46 ist so gewählt, dass sich auf der Außenoberfläche eine Oberflächenkontur 48 ausbildet, die durch die gewählte Querschnittsform der eingesetzten Wendeln 46 bestimmt ist. Am distalen Ende ist ein Folienventil 50 ausgebildet, das einen Durchfluss durch den Stent 40 nur vom proximalen Ende in Richtung distales Ende zulässt. Wird der Stent 40 vom proximalen Ende zum distalen Ende von einem Fluid oder Feststoff durchströmt, so öffnet sich das Folienventil und die Folienblätter öffnen sich, d.h., sie beabstanden sich voneinander. Findet keine Durchströmung des Stents 40 statt, so legen sich die Folienblätter aneinander an und verschließen das durch den Stent 40 gebildete Lumen an einem Ende.

In longitudinaler Richtung des Stents 40 ist in die Wandung des Stents 40 über die gesamte Länge ein Filament 51 eingearbeitet, das als Sicherungsfaden für die unterschiedlichen Abschnitte des Stents 40 dient. Das Filament 51 kann auch über den Stent 40 vorstehen, so dass mit dem Filament 51 der Stent 40 im Hohlorgan befestigt oder im Hohlorgan gezogen werden kann. Am proximalen Ende des Stents 40 ist, über den Umfang des Stents gesehen, ein Faden 52 als Fadenringanker eingearbeitet. Wird der Faden 52 zusammengezogen, so wird auch das Lumen des dritten Stentabschnitts 43 stark verkleinert, und der platzierte Stent 40 löst sich von der Innenoberfläche des Hohlorgans. Über den Faden 52 kann der Stent 40 aus einem Hohlorgan entfernt werden.

Ein Stent 10 für die transluminale Implantation weist zur Schienung und/oder zum Offenhalten eines Hohlorgans einen ersten, zweiten und dritten Stentabschnitt 11, 12, 13 auf, die über gewebefreie elastische tubuläre Abschnitte 14, 15 miteinander verbunden sind. Der Stent 10 vereint mindestens drei unterschiedliche Stent-Designs in einem Stent 10 und kann somit verbessert an das Bewegungsverhalten eines Hohlorgans angepasst werden.

## Patentansprüche

1. Stent (10; 20; 30;40) zur Schienung und/oder zum Offenhalten eines Hohlorgans, bestehend aus einem tubulären Körper, der einen Durchmesser aufweist, der durch axiale Verschiebung der Enden des Körpers relativ zueinander veränderbar ist, wobei der Körper mehrere im Querschnitt unterschiedliche voneinander unabhängige expandierbare Stentabschnitte (11, 12, 13; 21, 22, 23; 31, 32, 33; 41, 42, 43), die ein gewobenes, geflochtenes tubuläres Geflecht (16; 38) und/oder mindestens eine phasenverschobene Wendel (26; 36; 46) in mindestens einer Ebene aufweisen, aufweist,
**dadurch gekennzeichnet,**
**dass** die Stentabschnitte über gewebefreie elastische tubuläre Abschnitte (14, 15; 24, 25; 34, 35; 44, 45) miteinander verbunden sind,
wobei der Körper im belastungsfreien expandierten Zustand aus einem ersten Stentabschnitt (11; 21; 31; 41) mit einem ersten Durchmesser, einem zweiten und dritten Stentabschnitt (12, 13; 22, 23; 32, 33; 42, 43) mit einem zweiten und dritten Durchmesser und aus zwei gewebefreien elastischen tubulären Abschnitten (14, 15; 24, 25; 34, 35; 44, 45) gebildet ist, die den ersten, zweiten und dritten Stentabschnitt (11, 12, 13; 21, 22, 23; 31, 32, 33; 41, 42, 43) miteinander verbinden.

2. Stent nach Anspruch 1, **dadurch gekennzeichnet, dass** die Durchmesser der Stentabschnitte (11, 12, 13; 21, 22, 23; 31, 32, 33; 41, 42, 43) unterschiedlich sind.

3. Stent nach Anspruch 1, **dadurch gekennzeichnet, dass** bei mehreren Wendeln (36, 46) in mehreren Ebenen mindestens eine Wendel (36; 46) eine entgegengesetzte Windungsrichtung aufweist.

4. Stent nach einem der Ansprüche 1 bis 3, **dadurch gekennzeichnet, dass** die elastischen tubulären Abschnitte (14, 15; 24, 25; 34, 35; 44, 45) aus einem gewebeverträglichen Kunststoff hergestellt sind und/oder im belastungsfreien Zustand unterschiedliche Durchmesser aufweisen.

5. Stent nach einem der Ansprüche 1 bis 4, **dadurch gekennzeichnet, dass** der Körper, im Querschnitt gesehen, im ersten, zweiten und dritten Stentabschnitt (11, 12, 13; 21, 22, 23; 31, 32, 33; 41, 42, 43) ein Geflecht (16; 38) und/oder eine oder mehrere Wendeln (26; 36, 46) aufweist, die von einer oder mehreren elastischen Kunststoffschichten ummantelt sind, die materialschlüssig und geflecht- und wendelfrei in die elastischen tubulären Abschnitte (14, 15; 24, 25; 34, 35; 44, 45) übergehen.

6. Stent nach einem der Ansprüche 1 bis 5, **dadurch gekennzeichnet, dass** der Körper in axialer Richtung in der Wandung mindestens ein Filament (51) aufweist.

7. Stent nach einem der Ansprüche 1 bis 6, **dadurch gekennzeichnet, dass** im proximalen Endbereich des Körpers, über den Umfang des Körpers gesehen, ein Faden (52) eingearbeitet ist, dessen Länge über den Stent (40) hinausragt.

8. Stent nach Anspruch 1 bis 7, **dadurch gekennzeichnet, dass** am distalen Ende des Körpers ein Rückschlagventil, insbesondere ein Folienventil (50), ausgebildet ist.

9. Stent nach Anspruch 1, **dadurch gekennzeichnet, dass** die elastischen tubulären Abschnitte (14, 15; 24, 25; 34, 35; 44, 45) aus einer dünnen hochbeweglichen reissfesten Kunststofffolie, beispielsweise aus Silikon, gebildet sind.

## Claims

1. Stent (10; 20; 30; 40) for splinting and/or keeping open a hollow organ, consisting of a tubular body having a diameter which can be changed through axial displacement of the ends of the body relative to each other, wherein the body has several stent sections (11, 12, 13; 21, 22, 23; 31, 32, 33; 41, 42, 43) of different cross-sections, which can be expanded irrespectively of each other, comprise a woven, braided tubular web (16; 38) and/or at least one phase-shifted helix (26; 36; 46) in at least one plane, **characterized in that** the stent sections are connected to each other via web-free elastic tubular sections (14, 15; 24, 25; 34, 35; 44, 45), wherein the body is formed, in the unloaded expanded state, from a first stent section (11; 21; 31; 41) with a first diameter, a second and a third stent section (12, 13; 22, 23; 32, 33; 42, 43) having a second and a third diameter and from two web-free elastic tubular sections (14, 15; 24, 25; 34, 35; 44, 45) which connect the first, second and third stent sections (11, 12, 13; 21, 22, 23; 31, 32, 33; 41, 42, 43).

2. Stent according to claim 1, **characterized in that** the diameters of the stent sections (11, 12, 13; 21, 22, 23; 31, 32, 33; 41, 42, 43) are different.

3. Stent according to claim 1, **characterized in that** when several helices (36, 46) are provided in several planes, at least one helix (36; 46) has an opposite winding direction.

4. Stent according to any one of the claims 1 through 3, **characterized in that** the elastic tubular sections (14, 15; 24, 25; 34, 35; 44, 45) are produced from a tissue-compatible plastic material and/or have different diameters in the unloaded state.

5. Stent according to any one of the claims 1 through 4, **characterized in that** the body, viewed in cross-section, has a web (16; 38) in the first, second and third stent sections (11, 12, 13; 21, 22, 23; 31, 32, 3; 41, 42, 43) and/or one or more helices (26; 36, 46) which are surrounded by one or more elastic plastic layers which merge into the elastic tubular sections (14, 15; 24, 25; 34,35; 44, 45) in a material-bonding fashion without webs and helices.

6. Stent according to any one of the claims 1 through 5, **characterized in that** the body has at least one filament (51) in the wall in the axial direction.

7. Stent according to any one of the claims 1 through 6, **characterized in that** a thread (52) is worked into the proximal end region of the body, viewed over the periphery of the body, whose length projects past the stent (40).

8. Stent according to any one of the claims 1 through 7, **characterized in that** the distal end of the body comprises a return valve, in particular a foil valve (50).

9. Stent according to claim 1, **characterized in that** the elastic tubular sections (14, 15; 24, 25; 34, 35; 44, 45) are made from a thin, highly flexible and tear-proof plastic film, e.g. of silicon.

## Revendications

1. Stent (10 ; 20 ; 30 ; 40) destiné à éclisser et/ou maintenir ouvert un organe creux, formé par un corps tubulaire, dont le diamètre est variable sous l'effet du déplacement axial des extrémités du corps l'une par rapport à l'autre, le corps comportant plusieurs tronçons de stent (11, 12, 13 ; 21, 22, 23 ; 31, 32, 33 ; 41, 42, 43), qui ont une section différente et peuvent se dilater les uns indépendamment des autres et qui comportent dans au moins un plan un treillis tubulaire (16 ; 38) tissé, tressé et/ou au moins une spirale (26 ; 36 ; 46) décalée en phase, **caractérisé en ce que** les tronçons de stent sont assemblés entre eux par l'intermédiaire de tronçons tubulaires (14, 15 ; 24, 25 ; 34, 35 ; 44, 45) élastiques sans tissage, le corps étant formé à l'état dilaté, non soumis à sollicitation, par un premier tronçon de stent (11 ; 21 *;* 31 *;* 41) avec un premier diamètre, un deuxième et un troisième tronçon de stent (12, 13 ; 22, 23 ; 32, 33 *;* 42, 43) avec un deuxième et un troisième diamètre et par deux tronçons tubulaires (14, 15 *;* 24, 25 ; 34, 35 *;* 44, 45) élastiques sans tissage, qui relient entre eux le premier, le deuxième et le troisième tronçon de stent (11, 12, 13 ; 21, 22, 23 ; 31, 32, 33 ; 41, 42, 43).

2. Stent selon la revendication 1, **caractérisé en ce que** les diamètres des tronçons de stent (11, 12, 13 ; 21, 22, 23 ; 31, 32, 33 ; 41, 42, 43) sont différents.

3. Stent selon la revendication 1, **caractérisé en ce que**, en présence de plusieurs spirales (36, 46) dans plusieurs plans, au moins une spirale (36 ; 46) est enroulée dans une direction opposée.

4. Stent selon l'une quelconque des revendications 1 à 3, **caractérisé en ce que** les tronçons tubulaires (14, 15 ; 24, 25 ; 34, 35 ; 44, 45) élastiques sont réalisés dans une matière plastique apte à être tissée et/ou ont des diamètres différents à l'état non soumis à sollicitation.

5. Stent selon l'une quelconque des revendications 1 à 4, **caractérisé en ce que** le corps, par référence à une coupe transversale, comporte dans le premier, deuxième et troisième tronçon de stent (11, 12, 13 ; 21, 22, 23 ; 31, 32, 33 ; 41, 42, 43) un treillis (16 ; 38) et/ou une ou plusieurs spirales (26 ; 36 ; 46) qui sont enveloppées par une ou plusieurs couches élastiques en matière plastique, qui se prolongent par assemblage de matière et sans treillis et sans spirale pour former les tronçons tubulaires (14, 15 ; 24, 25 ; 34, 35 ; 44, 45) élastiques.

6. Stent selon l'une quelconque des revendications 1 à 5, **caractérisé en ce que** le corps comporte dans le sens axial au moins un filament (51) dans la paroi.

7. Stent selon l'une quelconque des revendications 1 à 6, **caractérisé en ce que** dans la zone d'extrémité proximale du corps, sur le pourtour du corps, est inséré un fil (52) dont la longueur s'avance hors du stent (40).

8. Stent selon l'une quelconque des revendications 1 à 7, **caractérisé en ce qu'**au niveau de l'extrémité distale du corps est réalisé un clapet anti-retour, en particulier une soupape à membrane (50).

9. Stent selon la revendication 1, **caractérisé en ce que** les tronçons tubulaires (14, 15 ; 24, 25 ; 34, 35 ; 44, 45) élastiques sont réalisés dans une mince feuille en matière plastique souple très rétractile et résistant à la déchirure, telle que la silicone.
